# EUROPÄISCHE PATENTSCHRIFT

(11) **EP 0 814 766 B1**
(45) Veröffentlichungstag und Bekanntmachung des Hinweises auf die Patenterteilung: **22.08.2001**
(21) Anmeldenummer: 96905790.0
(22) Anmeldetag: 24.02.1996
(51) Int. Cl.: A61K 7/50

(54) **KOSMETISCHE UND/ODER PHARMAZEUTISCHE ZUBEREITUNGEN**
COSMETIC AND/OR PHARMACEUTICAL PREPARATIONS
PREPARATIONS COSMETIQUES ET/OU PHARMACEUTIQUES

(30) Priorität: 03.03.1995 DE 19507531
(43) Veröffentlichungstag der Anmeldung: 07.01.1998
(73) Patentinhaber: Cognis Deutschland GmbH, 40589 Düsseldorf (DE)
(72) Erfinder: WEUTHEN, Manfred, D-42697 Solingen (DE); KAWA, Rolf, D-40789 Monheim (DE); GONDEK, Helga, D-40589 Düsseldorf (DE)
(86) Internationale Anmeldenummer: EP9600763
(87) Internationale Veröffentlichungsnummer: WO9627366

(56) Entgegenhaltungen:
- WO-A-92/05764
- DE-A- 4 331 297
- DE-A- 4 413 435
- DE-A- 4 424 823
- DE-C- 4 238 210
- DE-C- 4 238 211
- DE-C- 4 435 383

## Beschreibung

### Gebiet der Erfindung

Die Erfindung betrifft die Verwendung von Mischungen aus Fettsäure-N-alkylpolyhydroxyalkylamiden und Fettalkoholen als O/W-Emulgatoren in kosmetischen und/oder pharmazeutischen Zubereitungen.

### Stand der Technik

Zur Herstellung von kosmetischen Emulsionen wie beispielsweise Cremes und Lotionen sowie pharmazeutischen Zubereitungen wie etwa Salben besteht ein Bedarf an O/W-Emulgatoren, die sowohl die Einarbeitung polarer als auch unpolarer Öle gestatten. Eine Übersicht hierzu findet sich von G.Schuster et al. in **Ärztl.Kosmet. 11, 30 (1981).**

Die O/W-Emulgatoren des Stands der Technik sind jedoch in der Verwendung unbefriedigend, da sie entweder die Verwendung von unpolaren oder polaren Ölen erlauben, die Emulsionen eine unvorteilhaft niedrige Viskosität aufweisen, nicht ausreichend lager- bzw. viskositätsstabil sind und/oder die Emulsionen einen unerwünschten Gehalt an Ethylenoxideinheiten aufweisen.

Die internationale Patentanmeldung **WO 92/05764** offenbart die Verwendung von bestimmten Mischungen aus Glucamiden und Fettalkoholen als Tenside.

Die komplexe Aufgabe der Erfindung hat somit darin bestanden, kosmetische bzw. pharmazeutische Zubereitungen sowohl auf Basis polarer als auch unpolarer Öle zur Verfügung zu stellen, die sich durch eine verbesserte Viskosität und/oder Lager- bzw. Viskositätsstabilität auszeichnen und frei von ethylenoxidhaltigen Emulgatoren sind.

### Beschreibung der Erfindung

Gegenstand der Erfindung ist die Verwendung von Mischungen, enthaltend
(a) Fettsäure-N-alkylpolyhydroxyalkylamide der Formel **(I),** 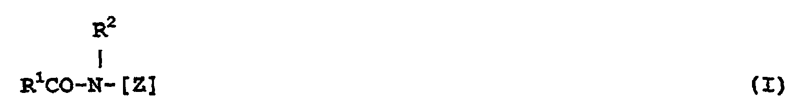 in der R¹CO für einen aliphatischen Acylrest mit 16 bis 22 Kohlenstoffatomen, R² für einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen, und
(b) Fettalkohole
im Gewichtsverhältnis (a) : (b) ≈ 75 : 25 bis 5 : 95 als O/W-Emulgatoren zur Herstellung von kosmetischen und/oder pharmazeutischen Zubereitungen.

Überraschenderweise wurde gefunden, daß die erfindungsgemäßen Fettsäure-N-alkylpolyhydroxyalkylamide, insbesondere aber Fettsäure-N-methylglucamide der angegebenen C-Kettenlänge, sowohl polare als auch unpolare Öle emulgieren und sich die Emulsionen durch eine vorteilhaft hohe und stabile Viskosität, Eleganz, Cremigkeit und Lagerstabilität auszeichnen. Besonders gute Ergebnisse werden erzielt, wenn man als Co-Emulgatoren hydrophile Wachse, vorzugsweise vom Typ der Fettalkohole insbesondere einer korrespondierenden C-Kettenlänge einsetzt.

### Fettsäure-N-alkylpolyhydroxyalkylamide

Fettsäure-N-alkylpolyhydroxyalkylamide, die im Sinne der Erfindung als O/W-Emulgatoren (Komponente a) eingesetzt werden können, folgen der Formel **(I)**, 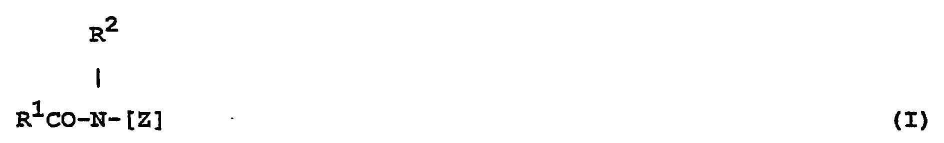 in der R¹CO für einen aliphatischen Acylrest mit 12 bis 22 Kohlenstoffatomen, R² für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften **US 1985424, US 2016962** und **US 2703798** sowie die Internationale Patentanmeldung **WO 92/06984** verwiesen. Eine Übersicht zu diesem Thema von H.Kelkenberg findet sich in **Tens. Surf.Det. 25, 8 (1988).**

Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher **Fettsäure-N-alkylglucamide** dar, wie sie durch die Formel **(II)** wiedergegeben werden:

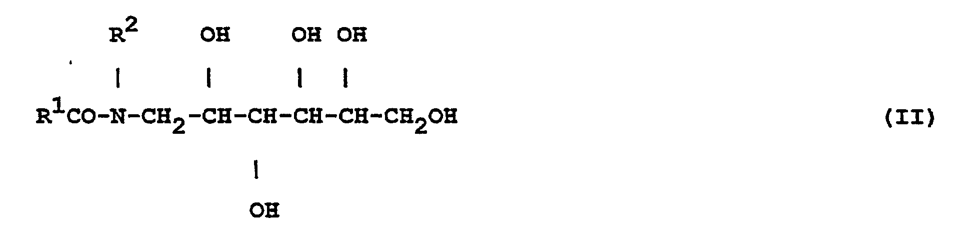

Vorzugsweise werden als Fettsäure-N-alkylpolyhydroxyalkylamide Glucamide der Formel **(II)** eingesetzt, in der R² für Wasserstoff oder eine Methylgruppe steht und R¹CO für den Acylrest der Palmitin- und/oder Stearinsäure steht. Besonders bevorzugt sind Fettsäure-N-alkylglucamide der Formel **(II),** die durch reduktive Aminierung von Glucose mit Methylamin und besonders vorteilhaften Ausführungsform der Erfindung empfiehlt es sich, Fettsäure-N-alkylpolyhydroxyalkylamide und Fettalkohole mit gleicher Zahl von Kohlenstoffatomen im Fettrest einzusetzen.

Im Sinne der Erfindung werden Fettalkohole (Komponente b) der Formel (III) eingesetzt,

**R**^{**3**}**OH (III)**

in der R³ für einen Alkyl- und/oder Alkenylrest mit 6 bis 22 Kohlenstoffatomen steht.

Die erfindungsgemäßen Zubereitungen können die Komponenten (a) und (b) im Gewichtsverhältnis 75 : 25 bis 5 : 95, vorzugsweise 75 : 25 bis 10 : 90 und insbesondere 75 : 25 bis 25 : 75 enthalten. Die Einsatzmenge der O/W-Emulgatoren, d.h. der Komponente (a) alleine oder in Abmischung mit der Komponente (b), kann 0,1 bis 10, vorzugsweise 1 bis 5 Gew.-% - bezogen auf die Zubereitungen - betragen.

### Gewerbliche Anwendbarkeit

Die im Sinne der Erfindung einzusetzenden Fettsäure-N-alkylpolyhydroxyalkylamide emulgieren sowohl unpolare als auch polare Öle. Die resultierenden Emulsionen zeichnen sich durch eine hohe Viskosität und Lagerstabilität aus.

Die erfindungsgemäßen Fettsäure-N-alkylpolyhydroxyalkylamide in Abmischung mit Fettalkoholen können zur Herstellung von kosmetischen und/oder pharmazeutischen Zubereitungen, wie z.B. Cremes, Lotionen, Salben und dergleichen verwendet werden. Die O/W-Emulgatoren können in den Mitteln in Mengen von 0,1 bis 10, vorzugsweise 1 bis 3 Gew.-%-bezogen auf die Mittel - enthalten sein.

Ferner können die Zubereitungen einen Gehalt an Ölkörpern, Co-Emulgatoren, Fetten und Wachsen, Stabilisatoren sowie Überfettungsmitteln, Verdickungsmitteln, biogenen Wirkstoffen, Filmbildnern, Konservierungsmitteln, Farb- und Duftstoffen aufweisen.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆- C₂₀-Fettalkoholen, Ester von verzweigten C₆-C₁₃-Carbonsäuren mit linearen C₁₀-C₁₈-Fettalkoholen, Ester von linearen C₁₀-C₁₈-Fettsäuren mit verzweigten Alkoholen, insbesondere 2-Ethylhexanol, Ester von linearen und/oder verzweigten Fettsäuren mit mehrwertigen Alkoholen wie z.B. Dimerdiol/Trimerdiol und/oder Guerbetalkoholen, Triglyceride auf Basis C₆-C₁₀-Fettsäuren, pflanzliche Öle, verzweigte primäre Alkohole, substituierte Cyclohexane, Guerbetcarbonate und/oder Dialkylether in Betracht.

Als **Co-Emulgatoren** kommen beispielsweise gehärtetes und ethoxyliertes Ricinusöl, Polyglycerinfettsäureester oder Polyglycerinpolyricinoleate bzw. -12-hydroxystearate sowie Fettalkoholsulfate, Seifen, Fettalkoholpolyglycolether, Alkyloligoglucoside, Partialglyceride, Sorbitanester und dergleichen in Frage.

Typische Beispiele für **Fette** sind Glyceride, als **Wachse** kommen u.a. Bienenwachs, Paraffinwachs oder Mikrowachse gegebenenfalls in Kombination mit hydrophilen Wachsen, z.B. Cetylstearylalkohol in Frage.

Als **Stabilisatoren** können Metallsalze von Fettsäuren wie z.B. Magnesium-, Aluminium und/oder Zinkstearat eingesetzt werden.

Als **Überfettungsmittel** können Substanzen wie beispielsweise polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen.

Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon, Tenside wie beispielsweise Fettalkoholethoxylate mit eingeengter Homologenverteilung oder Alkyloligoglucoside sowie Elektrolyte wie Kochsalz und Ammoniumchlorid.

Unter **biogenen Wirkstoffen** sind beispielsweise Pflanzenextrakte und Vitaminkomplexe zu verstehen.

Gebräuchliche **Filmbildner** sind beispielsweise Chitosan, mikrokristallines Chitosan, quaterniertes Chitosan, Polyvinylpyrrolidon, Vinylpyrrolidon-Vinyl-acetat-Copolymerisate, Polymere der Acrylsäurereihe, quaternäre Cellulose-Derivate, Kollagen, Hyaluronsäure bzw. deren Salze und ähnliche Verbindungen.

Als **Konservierungsmittel** eignen sich beispielsweise Phenoxyethanol, Formaldehydlösung, Parabene, Pentandiol oder Sorbinsäure.

Als **Perlglanzmittel** kommen beispielsweise Glycoldistearinsäureester wie Ethylenglycoldistearat, aber auch Fettsäuremonoglycolester in Betracht.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, veröffentlicht im Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% und der nicht wäßrige Anteil ("Aktivsubstanzgehalt") 20 bis 80, vorzugsweise 30 bis 70 Gew.-% - bezogen auf die Mittel - betragen. Die Herstellung der Mittel kann in an sich bekannter Weise, d.h. beispielsweise durch Heiß-, Kalt-, Heiß-Heiß/Kalt- bzw. PIT-Emulgierung erfolgen. Hierbei handelt es sich um ein rein mechanisches Verfahren, eine chemische Reaktion findet nicht statt.

Die folgenden Beispiele sollen den Gegenstand der Erfindung näher erläutern, ohne ihn darauf einzuschränken.

### Beispiele

Das Emulgiervermögen wurde in verschiedenen Cremes unter Verwendung von Ölkörpern unterschiedlicher Polarität und verschiedenen Emulgatoren getestet. Die Viskositäten wurden bei 20°C in einem Brookfield RVF-Viskosimeter (Spindel E, 4 UpM, mit Helipath) bestimmt. Die Rezepturen R1 und R2 sind erfindungsgemäß, die Rezepturen R3 bis R6 dienen zum Vergleich. Die Ergebnisse sind in Tabelle 1 zusammengefaßt (Prozentangaben als Gew.-%):

**Tabelle 1**

| Viskositätsmessungen | | | | | | |
|---|---|---|---|---|---|---|
| **Zusammensetzung** | **R1** | **R2** | **R3** | **R4** | **R5** | **R6** |
| | **%** | **%** | **%** | **%** | **%** | **%** |
| Myritol^{(R)} 318 | 16 | - | 16 | - | 16 | - |
| Paraffinöl | - | 16 | - | 16 | - | 16 |
| STMNMG | 2 | 2 | - | - | - | - |
| Emulgade^{(R)} PL 1618 | - | - | 2 | 2 | - | - |
| Eumulgin^{(R)} HRE 60 | - | - | - | - | 2 | 2 |
| Lanette^{(R)} O | 6 | 6 | 6 | 6 | 6 | 6 |
| Wasser, Formalin | ad 100 Gew.-% | | | | | |
| Viskosität¹⁾ [Pa*s] | 87 | 112 | 60 | 50 | 70 | 60 |
| Viskosität²⁾ [Pa*s] | 88 | 115 | 90 | 105 | 20 | 30 |

| | | | | | | |
|---|---|---|---|---|---|---|
| Legende: 1) Messung unmittelbar nach Herstellung | | | | | | |
| 2) Messung nach Lagerung von 1 Monat bei 25°C | | | | | | |

**Tabelle 2**

| Handelsnamen und CTFA-Bezeichnungen | | |
|---|---|---|
| **Handelsname** | | **CTFA-Bezeichnung** |
| Emulgade | PL 1618 | Cetearyl Polyglucose (and) |
| | | Cetearyl Alcohol |
| Eumulgin | HRE 60 | PEG-60 Hydrogenated Castor Oil |
| Lanette | O | Cetearyl Alcohol |
| Myritol | 318 | Caprylic/Capric Triglyceride |
| STSNMG | | Stearinsäure-N-methylglucamid* |

| | | |
|---|---|---|
| *) chemische Bezeichnung | | |

## Patentansprüche

1. Verwendung von Mischungen, enthaltend
(a) Fettsäure-N-alkylpolyhydroxyalkylamide der Formel **(I),** 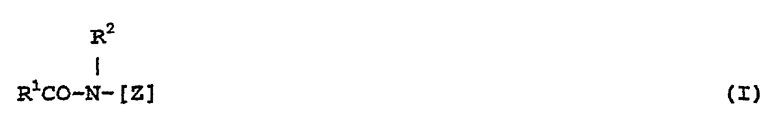 in der R¹CO für einen aliphatischen Acylrest mit 16 bis 22 Kohlenstoffatomen, R² für einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxyl-gruppen, und
(b) Fettalkohole
im Gewichtsverhältnis (a) : (b) = 75 : 25 bis 5 : 95 als O/W-Emulgatoren zur Herstellung von kosmetischen und/oder pharmazeutischen Zubereitungen.

2. Verwendung nach Anspruch 1, **dadurch gekennzeichnet,** daß man als Komponente (a) Fettsäure-N-alkylglucamide der Formel **(I)** einsetzt, in der R¹CO für einen aliphatischen Acylrest mit 16 bis 18 Kohlenstoffatomen steht.

3. Verwendung nach den Ansprüchen 1 und 2, **dadurch gekennzeichnet,** daß man als Komponente (b) Fettalkohole der Formel **(III)** einsetzt,
**R**^{**3**}**OH (III)**
in der R³ für einen Alkyl- und/oder Alkenylrest mit 6 bis 22 Kohlenstoffatomen steht.

4. Verwendung nach den Ansprüchen 1 bis 3, **dadurch gekennzeichnet,** daß man Komponenten (a) und (b) einsetzt, die im Fettrest gleiche Anzahlen von Kohlenstoffatomen aufweisen.

## Claims

1. The use of mixtures containing
(a) fatty acid-N-alkyl polyhydroxyalkylamides corresponding to formula (I) 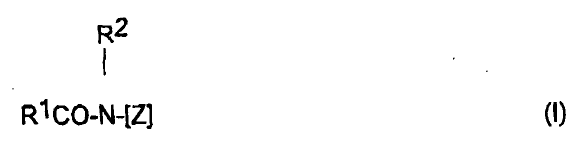 in which R¹CO is an aliphatic acyl group containing 16 to 22 carbon atoms, R² is an alkyl or hydroxyalkyl group containing 1 to 4 carbon atoms and [Z] is a linear or branched polyhydroxyalkyl group containing 3 to 12 carbon atoms and 3 to 10 hydroxyl groups and
(b) fatty alcohols
in a ratio by weight of (a) to (b) of 75:25 to 5:95 as o/w emulsifiers for the production of cosmetic and/or pharmaceutical preparations.

2. The use claimed in claim 1, characterized in that fatty acid-N-alkyl glucamides corresponding to formula (I), in which R¹CO is an aliphatic acyl group containing 16 to 18 carbon atoms, are used as component (a).

3. The use claimed in claims 1 and 2, characterized in that fatty alcohols corresponding to formula (III):
R³OH (II)
in which R³ is an alkyl and/or alkenyl group containing 6 to 22 carbon atoms, are used as component (b).

4. The use claimed in claims 1 to 3, characterized in that components (a) and (b) containing the same numbers of carbon atoms in the fatty residue are used.

## Revendications

1. Utilisation de mélanges contenant :
a) des N-alkylpolyhydroxyalkylamides d'aclde gras de formule I, 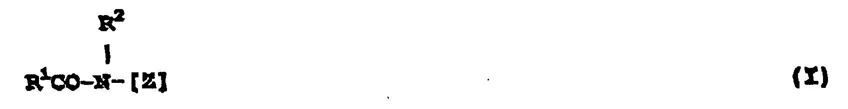 dans laquelle R¹CO représente un reste acyle aliphatique ayant de 16 à 22 atomes de carbone, R² représente un reste alkyle ou hydroxyalkyle ayant de 1 à 4 atomes de carbone et [Z] représente un reste polyhydroxyalkyle linéaire ou ramifié ayant de 3 à 12 atomes de carbone et de 3 à 10 groupes hydroxyle, et
b) des alcools gras
dans un rapport en poids (a) : (b) = 75 ; 25 à 5 : 95 comme agents émulsionnants H/E en vue de l'obtention de préparations cosmétiques et/ou pharmaceutiques.

2. Utilisation selon la revendication 1,
caractérisé en ce qu'
on met en oeuvre comme composants (a) des N-alkylglucamides d'acide gras de formule (1) dans laquelle R¹CO représente un reste acyle aliphatique ayant de 16 à 18 atomes de carbone.

3. Utilisation selon l'une quelconque des revendications 1 et 2,
caractérisée en ce qu'
on met en oeuvre comme composant (b) des alcools gras de formule (III),
R³OH (III)
dans laquelle R³ représente un reste alkyl et/ou alkényle ayant de 6 à 22 atomes de carbone.

4. Utilisation selon l'une quelconque des revendications 1 à 3,
caractérisée en ce qu'
on met en oeuvre des composants (a) et (b) qui possédent dans le reste gras, le même nombre d'atomes de carbone
